# EUROPEAN PATENT APPLICATION

(11) **EP 4 375 646 A1**
(43) Date of publication of application: **29.05.2024**
(21) Application number: 22902897.2
(22) Date of filing: 26.08.2022
(51) Int. Cl.: G01N 21/76, G01N 33/532, G01N 33/543, G01N 33/58, G01N 33/68

(54) **ZINC TRANSPORTER 8 ANTIBODY CHEMILUMINESCENCE IMMUNOASSAY KIT AND PREPARATION METHOD THEREOF**

(30) Priority: 09.12.2021 CN 202111498554
(71) Applicant: Shenzhen Yhlo Biotech Co., Ltd, 518116 Shenzhen (CN)
(72) Inventor: AN, Wenjun, Shenzhen, Guangdong 518116 (CN); LI, Huiping, Shenzhen, Guangdong 518116 (CN); ZHU, Liang, Shenzhen, Guangdong 518116 (CN); QIAN, Chungen, Shenzhen, Guangdong 518116 (CN)
(74) Representative: Michalski Hüttermann & Partner Patentanwälte mbB
(86) International application number: PCT/CN2022/115049
(87) International publication number: WO 2023/103464

(57) **Abstract**

Disclosed is a zinc transporter 8 antibody chemiluminescence immunoassay kit, comprising a zinc transporter 8 monomer antigen marked with a chemiluminescent group. The zinc transporter 8 monomer antigen is obtained by means of reducing a zinc transporter 8 multimeric antigen. The kit adopts a zinc transporter 8 monomer antigen marked with a chemiluminescence group for the detection of a zinc transporter 8 antibody. Because the zinc transporter 8 monomer antigen is obtained by means of reducing a zinc transporter 8 polymer antigen and has entirely exposed antigenic sites, which can be combined with more chemiluminescent groups, therefore the zinc transporter 8 monomer antigen marked with the chemiluminescence group has a high detection sensitivity.

## Description

### TECHNICAL FIELD

The present invention relates to the technical field of biomedical detection, specifically to a zinc transporter member 8 antibody chemiluminescence immunoassay kit and a preparation method thereof.

### BACKGROUND

Zinc transporter member 8 (ZnT8) is located in pancreatic beta cells in a dimer form and can transport cytoplasmic zinc ions into insulin storage/secretory vesicles. Reduced transport function of zinc transporter member 8 will affect the synthesis, storage, and secretion of insulin, and can increase the incidence risk of type 1 diabetes mellitus (T1DM). The ZnT8 protein can also serve as an antigen that causes autoimmune damage to beta cells and induces type 1 diabetes mellitus (T1DM). Type 1 diabetes mellitus is characterized by the presence of circulating autoantibodies, including islet cell antibodies (ICA), glutamic acid decarboxylase antibodies (GADA), tyrosine phosphatase antibodies (IA-2A), and human insulin antibodies (IAA), and zinc transporter member 8 antibodies (ZnT8A), in the serum of patients.

ZnT8A is a specific antibody for diagnosing type 1 diabetes mellitus, with a positive rate in initial cases of type 1 diabetes mellitus ranking second only to anti-GAD antibodies. Testing of ZnT8A can help assess the incidence risk of type 1 diabetes mellitus and has high diagnostic and prognostic value for assessing first-degree relatives of diabetic patients.

Currently, the commonly used clinical method for detecting zinc transporter member 8 antibodies is the magnetic particle chemiluminescence method, which uses the principle of the double-antigen sandwich method to detect and determine the relevant luminescence value (RLU). Chemiluminescence immunoassay (CLIA) is an immunoassay method that uses chemiluminescent agents to directly label antigens or antibodies. Chemiluminescent substances commonly used for labeling include acridinium ester (AE), which is an effective luminescent marker that emits light through the action of luminescence activating reagents (NaOH, H2O2), which is accomplished in one second, providing a quick flash of light.

However, the zinc transporter member 8 antigen has a complex molecular structure and often exists in the form of a polymer. Chemiluminescent labels are used to label the zinc transporter member 8 antigen. The zinc transporter member 8 antigen has low activity, poor reproducibility, large variation between batches, and a low labeling rate, resulting in low detection sensitivity.

### SUMMARY

In order to solve the above problems, a first object of the present invention is to provide a zinc transporter member 8 antibody chemiluminescence immunoassay kit. The kit allows the detection of zinc transporter member 8 antibodies by using a zinc transporter member 8 monomeric antigen labeled with a chemiluminescent group. Since the zinc transporter member 8 monomeric antigen has fully exposed antigenic sites so as to bind more chemiluminescent groups, the zinc transporter member 8 monomeric antigen labeled with chemiluminescent groups has higher detection sensitivity.

The present invention provides a zinc transporter member 8 antibody chemiluminescence immunoassay kit, including a zinc transporter member 8 monomeric antigen labeled with a chemiluminescent group. The zinc transporter member 8 monomeric antigen is obtained by reducing a zinc transporter member 8 polymeric antigen.

A second object of the present invention is to provide a method for preparing the above-mentioned zinc transporter member 8 antibody chemiluminescence immunoassay kit, including steps of preparing a zinc transporter member 8 monomeric antigen labeled with a chemiluminescent group:
reducing a zinc transporter member 8 polymeric antigen using a reducing agent to obtain a zinc transporter member 8 monomeric antigen; and
labeling the zinc transporter member 8 monomeric antigen with a chemiluminescent label to obtain the zinc transporter member 8 monomeric antigen labeled with the chemiluminescent label.

The present invention provides a zinc transporter member 8 antibody chemiluminescence immunoassay kit, which allows the detection of zinc transporter member 8 antibodies by using a zinc transporter member 8 monomeric antigen labeled with a chemiluminescent group. Since the zinc transporter member 8 monomeric antigen is produced by reduction of the zinc transporter member 8 polymeric antigen, the impact of the difference in polymer ratio is reduced, and the zinc transporter member 8 monomeric antigen has fully exposed antigenic sites so as to bind more chemiluminescent groups, which increase the labeling rate of the chemiluminescent label. Therefore, the zinc transporter member 8 monomeric antigen labeled with chemiluminescent groups has higher detection sensitivity.

### DETAILED DESCRIPTION

Reference now will be made in detail to the embodiments of the invention, one or more examples of which are set forth below. Each example is provided by way of explanation of the invention, not a limitation to the invention. In fact, it will be apparent to those skilled in the art that various modifications and variations can be made to the present invention without departing from the scope or spirit of the invention. For instance, features illustrated or described as part of one embodiment, can be used on another embodiment to yield a still further embodiment.

Thus, it is intended that the present invention covers such modifications and variations within the scope of the appended claims and their equivalents. Other objects, features and aspects of the present invention are disclosed in or are obvious from the following detailed description. It is to be understood by one of ordinary skill in the art that the present discussion is a description of exemplary embodiments only, and is not intended as limiting the broader aspects of the present invention.

The zinc transporter member 8 (ZnT8) antibodies are often conventionally detected using a magnetic particle chemiluminescence assay (CLIA), which is based on the following technical principle. To a sample of the zinc transporter member 8 antibody to be tested, zinc transporter member 8 antigen coated on a solid-phase carrier and zinc transporter member 8 antigen labeled with a chemiluminescent label are added in sequence and reacted to form a double-antigen sandwichantibody complex. A pre-excitation solution and an excitation solution are added to carry out a luminescence reaction. The content of zinc transporter member 8 antibody is calculated based on the luminescence intensity and standard curve.

At present, the zinc transporter member 8 antigen is generally labeled by directly labeling the ZnT8 antigen to be conjugated with acridinium ester, with the ZnT8 antigen often existing in the form of dimers. This labeling method has problems such as low labeling rate, poor stability and activity of the labeled ZnT8 antigen, poor reproducibility, and large variation between batches.

In order to at least partially solve at least one of the above technical problems, in a first aspect, the present invention provides a zinc transporter member 8 antibody chemiluminescence immunoassay kit, including a zinc transporter member 8 monomeric antigen labeled with a chemiluminescent group. The zinc transporter member 8 monomeric antigen is obtained by reducing the zinc transporter member 8 polymeric antigen.

In the present invention, a zinc transporter member 8 monomeric antigen labeled with a chemiluminescent group is creatively utilized to detect zinc transporter member 8 antibodies. Since the zinc transporter member 8 monomeric antigen is produced by reduction of the zinc transporter member 8 polymeric antigen, the zinc transporter member 8 monomeric antigen has fully exposed antigenic sites and thus higher activity so as to bind more chemiluminescent groups, which increase the labeling rate of the chemiluminescent label. Therefore, the zinc transporter member 8 monomeric antigen labeled with chemiluminescent groups has higher detection sensitivity.

In some embodiments, the zinc transporter member 8 monomeric antigen is further modified with a blocking group. The blocking group is attached to the zinc transporter member 8 monomeric antigen and plays a blocking role to prevent the zinc transporter member 8 monomeric antigen from repolymerizing, which will affect the activity of the zinc transporter member 8 monomeric antigen and reduce the binding efficiency of the zinc transporter member 8 monomeric antigen to the chemiluminescent group.

In some embodiments, the blocking group is a thiol group-blocking group. The thiol group-blocking group is attached to a thiol group on the zinc transporter member 8 monomeric antigen and plays a blocking role to prevent the zinc transporter member 8 monomeric antigen from repolymerizing by the thiol group, which will affect the activity of the zinc transporter member 8 monomeric antigen and reduce the binding efficiency of the zinc transporter member 8 monomeric antigen to the chemiluminescent group.

In some embodiments, the thiol group-blocking group is at least one derived from N-ethylmaleimide (NEM), N-hydroxymaleimide, and iodoacetamide. It can be understood that the agent from which the blocking group is derived is not limited by the range of blocking agents listed above. Any substance that can bind to a thiol group to block the thiol group without affecting subsequent chemiluminescence immunoassay can be used as a blocking agent to provide a blocking group.

In some embodiments, the chemiluminescent group is at least one derived from acridine ester (AE-NHS, DMAE-NHS), acrinic acid (9-acridinecarboxylic acid), acridine amide or acridine sulfonamide (NSP-SA-NHS). The chemiluminescent group is attached to an amino group on the zinc transporter member 8 monomeric antigen and can emit light quickly under the excitation of the excitation solution or pre-excitation solution, allowing the content of zinc transporter member 8 antibody to be calculated based on the luminescence intensity and standard curve.

In a second aspect, the present invention provides a method for preparing the above-mentioned zinc transporter member 8 antibody chemiluminescence immunoassay kit, including steps of preparing a zinc transporter member 8 monomeric antigen labeled with a chemiluminescent group:
reducing a zinc transporter member 8 polymeric antigen using a reducing agent to obtain a zinc transporter member 8 monomeric antigen; and
labeling the zinc transporter member 8 monomeric antigen with a chemiluminescent label to obtain the zinc transporter member 8 monomeric antigen labeled with the chemiluminescent group.

Specifically, a zinc transporter member 8 polymeric antigen is diluted in PBS buffer to prepare a solution of zinc transporter member 8 polymeric antigen. To the solution of ZnT8 polymeric antigen, a reducing agent is added to a working concentration, and then a reduction reaction is carried out for a period of time. After the reaction is completed, the reaction product was desalted in a desalting column that has been equilibrated with PBS for removal of impurities from the reaction product, to obtain a purified solution of ZnT8 monomeric antigen.

Further, a chemical label dissolved in DMSO solvent is added to and reacted with the solution of ZnT8 monomeric antigen for labeling. Using TRIS (pH 7.4) buffer as exchange buffer, the reaction product is passed through a desalting column with a molecular weight cutoff to remove free chemical labels and reaction by-products, and to obtain a solution of ZnT8 monomeric antigen labeled with the chemiluminescent group.

It can be understood that through the labeling method of the present invention, a ZnT8 monomeric antigen prepared by reducing the zinc transporter member 8 polymeric antigen can have improved activity, so that the ZnT8 antigen can have more exposed active sites, and thus can increase the labeling rate of a chemiluminescent marker when labeling the ZnT8 monomeric antigen with the chemiluminescent label. When using the ZnT8 monomeric antigen labeled with the chemiluminescent label to detect ZnT8 monomer antibodies, the sensitivity for detecting the antibodies can be improved.

In some embodiments, a mole number of the chemiluminescent label is 1-5000 times a mole number of the zinc transporter member 8 monomeric antigen, for example, can be 5-4000 times, 10-3000 times, 20-2000 times the mole number of the zinc transporter member 8 monomeric antigen, and further can be 30-1000 times, 50-400 times, 100-300 times, or 200 times, 1500 times, 2500 times, 3500 times, 4500 times the mole number of the zinc transporter member 8 monomeric antigen.

In some embodiments, the reducing agent includes at least one of dithiothreitol, mercaptoethylamine, mercaptoethanol, and tricarboxyethylphosphine.

In some embodiments, a mole number of the reducing agent is 1-2500 times the mole number of zinc transporter member 8 polymeric antigen, for example, can be 5-2000 times, 10-1500 times, 20-1000 times the mole number of zinc transporter member 8 polymeric antigen, and further can be 30 -600 times, 40-300 times, or 50 times, 100 times, 200 times the mole number of zinc transporter member 8 polymeric antigen.

In some embodiments, before or after labeling the zinc transporter member 8 monomeric antigen with the chemiluminescent label, the method further includes: blocking the zinc transporter member 8 monomeric antigen with a blocking agent to obtain a thiol group-blocked acridinium ester-labeled zinc transporter member 8 monomeric antigen, so that the zinc transporter member 8 monomeric antigen is attached with a blocking group through a chemical reaction.

Specifically, to the ZnT8 monomeric antigen solution, a blocking agent is added to a working concentration, and reacted for a period of time at room temperature. After the reaction is completed, the reaction product was desalted in a desalting column that has been equilibrated with PBS for removal of impurities from the reaction product, to obtain a purified solution of thiol group-blocked ZnT8 monomeric antigen. It can be understood that the step of blocking the thiol group can be performed before or after labeling the ZnT8 monomeric antigen.

In some embodiments, a mole number of the blocking agent is 1-2000 times, preferably 20-200 times, the mole number of zinc transporter member 8 polymeric antigen.

The embodiments of the present invention will be described in detail below with reference to the examples.

### Example 1

This example provides a zinc transporter member 8 antibody chemiluminescence immunoassay kit including a thiol group-blocked acridinium ester-labeled zinc transporter member 8 monomeric antigen, and a preparation method thereof. The method includes the following steps.

### 1. Preparation of a solution of acridinium ester-labeled zinc transporter member 8 monomeric antigen

(1) Zinc transporter member 8 was diluted to 0.5 mg/mL with 0.05M PBS buffer to prepare a polymeric antigen solution.
(2) To the polymeric antigen solution in step (1), dithiothreitol (DTT) was added to a final concentration of 1.5 mg/mL and gently mixed. The mixture was reacted at room temperature (25°C) for 60 min. The reaction product was desalted in a desalting column that has been equilibrated with PBS for removal of impurities, to obtain a purified solution of zinc transporter 8 monomeric antigen.
(3) To the purified solution of zinc transporter 8 monomeric antigen obtained in step (2), N-ethylmaleimide (NEM) was added to a final concentration of 0.5 mg/Ml and gentlely mixed. The mixture was reacted at room temperature (25°C) for 60 min. The reaction product was desalted in a desalting column that has been equilibrated with PBS for removal of impurities, to obtain a purified solution of thiol group-blocked zinc transporter 8 monomeric antigen.
(4) 10 µL of 6.0 mg/mL acridinium ester dissolved in DMSO solvent was added to the solution of zinc transporter monomeric antigen, and the mixture was reacted at 25°C for 60 minutes. Using 50 mM TRIS (pH 7.4) buffer as exchange buffer, the reaction product was passed through a 2 mL desalting column with 7 KD molecular weight cutoff (Thermo Fisher) three times for removal of free acridinium ester and reaction by-products, to obtain a thiol group-blocked acridinium ester-labeled zinc transporter member 8 monomeric antigen.

### 2. Preparation of nanomagnetic beads coated with zinc transporter member 8 monomeric antigen

50 mg of a suspension of carboxylated magnetic particles (with particle size of 0.05-1 µm) was weighed, subjected to magnetic separation to remove the supernatant, and resuspended in 0.02 M MES buffer, pH 5.5. 0.5-2 mL of freshly prepared EDC aqueous solution at 10 mg/mL was added to activate the carboxyl groups on the surface of the magnetic beads. 3-5 mg of zinc transporter member 8 was added, suspended at room temperature for 2-10 h, magnetically separated to remove the supernatant, and then resuspended to 1 mg/mL in 0.1 M Tris buffer, pH 8.0 containing 2% BSA to obtain zinc transporter member 8-coated magnetic particles, which were divided into 5 mL aliquots per vial and stored at 4°C for later use.

### 3. Preparation of calibration products for zinc transporter member 8 antibody

Zinc transporter member 8 antibody was prepared at a series of concentration of 5AU/mL, 20AU/mL, 80AU/mL, 200AU/mL, 800AU/mL, and 2000AU/mL in a standard buffer (40 mM Tris-HCl, 0.5% BSA, 1% NaCl, pH 8.0), divided into 5 mL aliquots per vial for lyophilization, and stored at 4°C for later use.

### 4. Preparation of a chemiluminescent pre-excitation solution

In 1.0 liters of purified water, 80 µL of 20% by mass hydrogen peroxide (H₂O₂), 1.0 g of sodium azide, and 1.5 g of Tween 20 were added in sequence, shaken well, and stored in the dark.

### 5. Preparation of a chemiluminescent excitation solution

In 1.0 liters of purified water, 0.6 g of sodium hydroxide, 0.5 g of PC300, 0.5 g of sodium azide, and 1.5 g of Triton 405 were added in sequence, shaken well, and stored in the dark.

### Example 2

This example provides a zinc transporter member 8 antibody chemiluminescence immunoassay kit including a thiol group-blocked acridinium ester-labeled zinc transporter member 8 monomeric antigen, and a preparation method thereof. The method includes the following steps.

### 1. Preparation of a solution of acridinium ester-labeled zinc transporter member 8 monomeric antigen

(1) Zinc transporter member 8 antigen was diluted to 0.5 mg/mL with 0.05M PBS buffer to prepare a solution of zinc transporter member 8 polymeric antigen.
(2) To the zinc transporter member 8 antigen solution in step (1), dithiothreitol (DTT) was added to a final concentration of 1.5 mg/mL and gently mixed. The mixture was reacted at room temperature (25°C) for 60 min. The reaction product was desalted in a desalting column that has been equilibrated with PBS for removal of impurities, to obtain a purified solution of zinc transporter 8 monomeric antigen.
(3) 10 µL of 6.0 mg/mL acridinium ester dissolved in DMSO solvent was added to the solution of antigen, and the mixture was reacted at 25°C for 60 minutes. Using 50 mM TRIS (pH 7.4) buffer as exchange buffer, the reaction product was passed through a 2 mL desalting column with 7 KD molecular weight cutoff (Thermo Fisher) three times for removal free acridinium ester and reaction by-products, to obtain a solution of acridinium ester-labeled zinc transporter member 8 monomeric antigen.
(4) To the solution of the acridinium ester-antigen conjugated product in step (3), N-ethylmaleimide (NEM) was added to a final concentration of 0.5 mg/mL and gently mixed. The mixture was reacted at room temperature (25°C) for 60 min. The reaction product was desalted in a desalting column that has been equilibrated with PBS for removal of impurities, to obtain a purified solution of thiol group-blocked acridinium ester-labeled zinc transporter 8 monomeric antigen.

### 2. Preparation of nanomagnetic beads coated with zinc transporter member 8 monomeric antigen

50 mg of a suspension of carboxylated magnetic particles (with particle size of 0.05 - 1 µm) was weighed, subjected to magnetic separation to remove the supernatant, and resuspended in 0.02 M MES buffer, pH 5.5. 0.5-2 mL of freshly prepared EDC aqueous solution at 10 mg/mL was added to activate the carboxyl groups on the surface of the magnetic beads. 3-5 mg of zinc transporter member 8 monomeric antigen was added, suspended at room temperature for 2-10 h, magnetically separated to remove the supernatant, and then resuspended to 1 mg/mL in 0.1 M Tris buffer, pH 8.0 containing 2% BSA to obtain thiol group-blocked acridinium ester-labeled zinc transporter member 8 monomeric antigen-coated magnetic particles, which were divided into 5 mL aliquots per vial and stored at 4°C for later use.

### 3. Preparation of calibration products for zinc transporter member 8 antibody

Zinc transporter member 8 antibody was prepared at a series of concentration 5AU/mL, 20AU/mL, 80AU/mL, 200AU/mL, 800AU/ mL, and 2000AU/mL in a standard buffer (40 mM Tris-HCl, 0.5% BSA, 1% NaCl, pH 8.0), divided into 5 mL aliquots per vial for lyophilization, and stored at 4°C for later use.

### 4. Preparation of a chemiluminescent pre-excitation solution

In 1.0 liters of purified water, 80 µL of 20% by mass hydrogen peroxide (H₂O₂), 1.0 g of sodium azide, and 1.5 g of Tween 20 were added in sequence, shaken well, and store in the dark.

### 5. Preparation of a chemiluminescent excitation solution

In 1.0 liters of purified water, 0.6 g of sodium hydroxide, 0.5 g of PC300, 0.5 g of sodium azide, and 1.5 g of Triton 405 were added in sequence, shaken well, and store in the dark.

### Comparative Example 1

This example provides a zinc transporter member 8 antibody chemiluminescence immunoassay kit including an acridinium ester-labeled zinc transporter member 8 polymeric antigen, and a preparation method thereof. The method includes the following steps.

### 1. Preparation of a solution of acridinium ester-labeled zinc transporter member 8 antigen

(1) zinc transporter member 8 antigen was diluted to 0.5 mg/mL with 0.05M PBS buffer to prepare an antigen solution.
(2) 10 µL of 6.0 mg/mL acridinium ester dissolved in DMSO solvent was added to the solution of antigen, and the mixture was reacted at 25°C for 60 minutes. Using 50 mM TRIS (pH 7.4) buffer as exchange buffer, the reaction product was passed through a 2 mL desalting column with 7 KD molecular weight cutoff (Thermo Fisher) three times for removal free acridinium ester and reaction by-products, to obtain a solution of acridinium ester-labeled zinc transporter member 8 antigen.

### 2. Preparation of nanomagnetic beads coated with zinc transporter member 8 antigen

50 mg of a suspension of carboxylated magnetic particles (with particle size of 0.05-1 µm) was weighed, subjected to magnetic separation to remove the supernatant, and resuspended in 0.02 M MES buffer, pH 5.5. 0.5-2 mL of freshly prepared EDC aqueous solution at 10 mg/mL was added to activate the carboxyl groups on the surface of the magnetic beads. 3-5 mg of zinc transporter member 8 monomeric antigen was added, suspended at room temperature for 2-10 h, magnetically separated to remove the supernatant, and then resuspended to 1 mg/mL in 0.1 M Tris buffer, pH 8.0 containing 2% BSA to obtain zinc transporter member 8 antigen-coated magnetic particles, which were divided into 5 mL aliquots per vial and stored at 4°C for later use.

### 3. Preparation of calibration products for zinc transporter member 8 antibody

Zinc transporter member 8 antibody was prepared at a series of concentration of 5AU/mL, 20AU/mL, 80AU/mL, 200AU/mL, 800AU/ mL, and 2000AU/Ml in a standard buffer (40 mM Tris-HCl, 0.5% BSA, 1% NaCl, pH 8.0), divided into 5 mL aliquots per vial for lyophilization, and stored at 4°C for later use.

### 4. Preparation of a chemiluminescent pre-excitation solution

In 1.0 liters of purified water, 80 µL of 20% by mass hydrogen peroxide (H₂O₂), 1.0 g of sodium azide, and 1.5 g of Tween 20 were added in sequence, shaken well, and stored in the dark.

### 5. Preparation of a chemiluminescent excitation solution

In 1.0 liters of purified water, 0.6 g of sodium hydroxide, 0.5 g of PC300, 0.5 g of sodium azide, and 1.5 g of Triton 405 were added in sequence, shaken well, and stored in the dark.

### II. Chemiluminescence immunoassay of zinc transporter member 8 antibody

In this example, a fully automatic chemiluminescence immunoassay analyzer was used as the detection instrument, and the detection method was a double-antigen sandwich method. Specifically, 25 µL of sample, 50 µL of zinc transporter member 8-coated magnetic particles, and 50 µL of acridinium ester-labeled zinc transporter member 8 or thiol group-blocked acridinium ester-labeled zinc transporter member 8 monomeric antigen were added in sequence to the instrument. After reacting for 10 minutes, magnetic separation was performed. The reaction mixture was sent to the darkroom in the instrument, and 50 µL of the chemiluminescent pre-excitation solution and 50 µL of the chemiluminescent excitation solution were added in sequence for the luminescence reaction. Finally, the luminescence intensity was recorded, and the content of zinc transporter member 8 antibody in the tested sample was calculated from the standard curve.

### III. Performance evaluation of zinc transporter member 8 antibody chemiluminescence immunoassay kit

### 1. Sensitivity testing

The samples at the same concentration were subjected to zinc transporter member 8 antibody chemiluminescence immunoassay by using kits in Examples 1, 2 and Comparative Example 1. The sensitivity was characterized by the detection signal value. Under the same sample concentration, the higher the signal value, the higher the sensitivity. The specific test results are shown in Table 1. From Table 1, it can be seen that when the concentration was within the range of 6.83 to 285.93 AU/mL, the sensitivity of the kits of Example 1 and Example 2 was significantly greater than that of the kit of Comparative Example 1.

**Table 1**

| Concentration of sample | Signal value | | |
|---|---|---|---|
| AU/mL | Example 1 | Example 2 | Comparative Example 1 |
| 6.83 | 6085 | 5943 | 4881 |
| 7.02 | 9611 | 9386 | 5930 |
| 13.87 | 19414 | 18961 | 11979 |
| 25.67 | 30007 | 29307 | 18515 |
| 26.31 | 39353 | 38435 | 24282 |
| 54.26 | 62634 | 61172 | 38647 |
| 109.61 | 127030 | 124066 | 78381 |
| 115.31 | 157725 | 154045 | 97321 |
| 285.93 | 310106 | 302869 | 191344 |

### 2. Specificity testing

The kits in Examples 1, 2 and Comparative Example 1 were each used for testing the samples with or without ZnT8A. Whether the samples contained ZnT8A or not was determined based on the test results. Specificity is represented by the percentage of individuals who are correctly determined as negative to individuals who are actually disease-free. That is, the specificity is characterized by the accurate rate for testing negative samples. A higher accurate rate indicates that the result has more accuracy and thus better specificity. The specific test results are shown in Table 2. From Table 2, it can be seen that the specificity of the kits in Example 1 and Example 2 is significantly higher than that of the kit of Comparative Example 1.

**Table 2**

| | Example 1 | Example 2 | Example 3 |
|---|---|---|---|
| Positive | 115 | 116 | 116 |
| Negative | 3 | 2 | 10 |
| Total | 118 | 118 | 126 |
| Specificity | 96.9% | 98.0% | 89.8% |

### 3. Stability testing

The antigen solutions in the kits prepared in Examples 1, 2 and Comparative Example 1 were each placed in incubators at 4°C and 37°C for 7 days. The samples at different concentrations were tested for chemiluminescence signal values. The antigen solution at 4°C was used as a control to calculate the stability of the solution accelerated for 7 days at 37°C. A smaller absolute value of the stability deviation indicates better stability of the working solution. The specific test results are shown in Table 3, Table 4 and Table 5. From Tables 3, 4 and 5, it can be seen that the stability of the antigen solutions in the kits of Example 1 and Example 2 is significantly higher than that of the antigen solution in the kit of Comparative Example 1.

**Table 3**

| Concentration of sample in AU/mL | Signal value of Example 1 | | Relative deviation |
|---|---|---|---|
| | 4°C control | 37°C acceleration | |
| 6.83 | 6008 | 5538 | -7.8% |
| 7.02 | 9568 | 8841 | -7.6% |
| 13.87 | 19017 | 18458 | -2.9% |
| 25.67 | 29946 | 28090 | -6.2% |
| 26.31 | 39234 | 36961 | -5.8% |
| 54.26 | 61525 | 55555 | -9.7% |
| 109.61 | 126008 | 118967 | -5.6% |
| 115.31 | 156509 | 152551 | -2.5% |
| 285.93 | 306344 | 282638 | -7.7% |

**Table 4**

| Concentration of sample in AU/mL | Signal value of Example 2 | | Relative deviation |
|---|---|---|---|
| | 4°C control | 37°C acceleration | |
| 6.83 | 6048 | 5608 | -7.3% |
| 7.02 | 9606 | 9307 | -3.1% |
| 13.87 | 19112 | 17762 | -7.1% |
| 25.67 | 29489 | 26997 | -8.4% |
| 26.31 | 38983 | 36519 | -6.3% |
| 54.26 | 61645 | 55595 | -9.8% |
| 109.61 | 124111 | 114986 | -7.4% |
| 115.31 | 156161 | 145965 | -6.5% |
| 285.93 | 303060 | 286921 | -5.3% |

**Table 5**

| Concentration of sample in AU/mL | Signal value of Comparative Example 1 | | Relative deviation |
|---|---|---|---|
| | 4°C control | 37°C acceleration | |
| 6.83 | 5043 | 4468 | -11.4% |
| 7.02 | 6109 | 5312 | -13.0% |
| 13.87 | 12925 | 10755 | -16.8% |
| 25.67 | 22137 | 19540 | -11.7% |
| 26.31 | 27204 | 23085 | -15.1% |
| 54.26 | 41775 | 36229 | -13.3% |
| 109.61 | 84215 | 72971 | -13.4% |
| 115.31 | 98333 | 84971 | -13.6% |
| 285.93 | 195259 | 174132 | -10.8% |

### 4. Difference between batches

Three different batches of antigens were labeled with acridine to prepare the kits of Example 1, Example 2 and Comparative Example 1. The chemiluminescence signal values of samples at different concentrations were tested for determining the difference in the kits prepared from different batches of raw materials. The first batch of kits was used as a control to calculate the relative deviations of the second and third batches. A smaller relative deviation indicates a smaller variation between the batches. The results are shown in Tables 6, 7 and 8 below. From the data in Tables 6, 7 and 8, it can be seen that the variation between the different batches of the kits prepared in Example 1 and Example 2 is much smaller than the variation between the different batches of the kit of Comparative Example 1.

**Table 6**

| Concentration of sample in AU/mL | Signal value of Example 1 | | | Relative deviation | |
|---|---|---|---|---|---|
| | First batch | Second batch | Third batch | Relative deviation between the first batch and the second batch | Relative deviation between the first batch and the third batch |
| 6.83 | 5775 | 6234 | 5984 | 7.9% | 3.6% |
| 7.02 | 9108 | 9932 | 9465 | 9.0% | 3.9% |
| 13.87 | 18544 | 20355 | 19085 | 9.8% | 2.9% |
| 25.67 | 28928 | 30590 | 31171 | 5.7% | 7.8% |
| 26.31 | 38554 | 40498 | 38957 | 5.0% | 1.0% |
| 54.26 | 61796 | 64442 | 64793 | 4.3% | 4.8% |
| 109.61 | 126317 | 130230 | 129138 | 3.1% | 2.2% |
| 115.31 | 156803 | 160197 | 160196 | 2.2% | 2.2% |
| 285.93 | 303070 | 315676 | 310266 | 4.2% | 2.4% |

**Table 7**

| Concentration of sample in AU/mL | Signal value of Example 2 | | | Relative deviation | |
|---|---|---|---|---|---|
| | First batch | Second batch | Third batch | Relative deviation between the first batch and the second batch | Relative deviation between the first batch and the third batch |
| 6.83 | 5662 | 6204 | 6054 | 9.6% | 6.9% |
| 7.02 | 8980 | 9761 | 9575 | 8.7% | 6.6% |
| 13.87 | 18594 | 20044 | 20179 | 7.8% | 8.5% |
| 25.67 | 29051 | 30636 | 30175 | 5.5% | 3.9% |
| 26.31 | 38640 | 39675 | 40177 | 2.7% | 4.0% |
| 54.26 | 61183 | 65105 | 64644 | 6.4% | 5.7% |
| 109.61 | 126043 | 127813 | 133046 | 1.4% | 5.6% |
| 115.31 | 154150 | 156358 | 163038 | 1.4% | 5.8% |
| 285.93 | 306457 | 323362 | 330340 | 5.5% | 7.8% |

**Table 8**

| Concentration of sample in AU/mL | Signal value of Comparative Example 1 | | | Relative deviation | |
|---|---|---|---|---|---|
| | First batch | Second batch | Third batch | Relative deviation between the first batch and the second batch | Relative deviation between the first batch and the third batch |
| 6.83 | 4950 | 5601 | 5421 | 13.2% | 9.5% |
| 7.02 | 5964 | 6912 | 7048 | 15.9% | 18.2% |
| 13.87 | 11872 | 14004 | 13989 | 18.0% | 17.8% |
| 25.67 | 17549 | 20463 | 21158 | 16.6% | 20.6% |
| 26.31 | 24681 | 28444 | 27138 | 15.2% | 10.0% |
| 54.26 | 37920 | 44041 | 44609 | 16.1% | 17.6% |
| 109.61 | 78542 | 87091 | 86421 | 10.9% | 10.0% |
| 115.31 | 97231 | 108760 | 108851 | 11.9% | 12.0% |
| 285.93 | 193144 | 222386 | 228574 | 15.1% | 18.3% |

The technical features of the above embodiments can be combined arbitrarily. To simplify the description, not all possible combinations of the technical features in the above embodiments are described. However, all of the combinations of these technical features should be considered as within the scope of this disclosure, as long as such combinations do not contradict with each other.

The aforementioned examples merely represent several embodiments of the present invention, and the description thereof is more specific and detailed, but it should not be construed as limiting the scope of the present invention. It should be noted that, several modifications and improvements may be made for those of ordinary skill in the art without departing from the concept of the present invention, and these are all within the protection scope of the present invention. Therefore, the protection scope of the present disclosure shall be subject to the appended claims.

## Claims

1. A zinc transporter member 8 antibody chemiluminescence immunoassay kit, comprising a zinc transporter member 8 monomeric antigen labeled with a chemiluminescent group, wherein the zinc transporter member 8 monomeric antigen is obtained by reducing a zinc transporter member 8 polymeric antigen.

2. The kit according to claim 1, wherein the zinc transporter member 8 monomeric antigen is further modified with a blocking group, and the blocking group is attached to the zinc transporter member 8 monomeric antigen via a chemical bond.

3. The kit according to claim 2, wherein the blocking group is a thiol group-blocking group, and the blocking group is attached to a thiol group on the zinc transporter member 8 monomeric antigen via a chemical bond.

4. The kit according to claim 3, wherein the chemiluminescent group is at least one derived from acridine ester, acrinic acid, acridine amide and acridine sulfonamide; and
the thiol group-blocking group is at least one derived from N-ethylmaleimide, N-hydroxymaleimide, and iodoacetamide.

5. A method for preparing a kit of any one of claims 1-4, comprising the steps of:
reducing a zinc transporter member 8 polymeric antigen using a reducing agent to obtain a zinc transporter member 8 monomeric antigen; and
labeling the zinc transporter member 8 monomeric antigen with a chemiluminescent label to obtain the zinc transporter member 8 monomeric antigen labeled with the chemiluminescent group.

6. The method according to claim 5, wherein a mole number of the chemiluminescent label is 1-5000 times a mole number of the zinc transporter member 8 monomeric antigen.

7. The method according to claim 5, wherein the reducing agent comprises at least one of dithiothreitol, mercaptoethylamine, mercaptoethanol, and tricarboxyethylphosphine.

8. The method according to claim 7, wherein a mole number of the reducing agent is 1-2500 times a mole number of zinc transporter member 8 polymeric antigen.

9. The method according to claim 7, wherein before or after labeling the zinc transporter member 8 monomeric antigen with the chemiluminescent label, the method further comprises:
blocking the zinc transporter member 8 monomeric antigen with a blocking agent to obtain a thiol group-blocked acridinium ester-labeled zinc transporter member 8 monomeric antigen, so that the zinc transporter member 8 monomeric antigen is attached with a blocking group through a chemical reaction.

10. The method according to claim 9, wherein a mole number of the blocking agent is 1-2000 times a mole number of zinc transporter member 8 polymeric antigen.
